# EUROPEAN PATENT SPECIFICATION

(11) **EP 1 785 484 B1**
(45) Date of publication and mention of the grant of the patent: **01.06.2011**
(21) Application number: 05750939.0
(22) Date of filing: 20.06.2005
(51) Int. Cl.: C12N 15/09, A61K 31/7105, A61K 31/713, A61K 48/00, A61P 35/00, C07H 21/02

(54) **OLIGONUCLEOTIDE INHIBITING TUMOR CELL PROLIFERATION AND METHOD THEREFOR**
TUMORZELLPROLIFERATION HEMMENDES OLIGONUKLEOTID UND VERFAHREN DAFÜR
OLIGONUCLEOTIDE INHIBANT LA PROLIFERATION DE CELLULES DE TUMEUR ET PROCEDE POUR CELA

(30) Priority: 30.06.2004 JP 2004192718
(43) Date of publication of application: 16.05.2007
(73) Proprietor: Japan Science and Technology Agency, Kawaguchi-shi, Saitama 332-0012 (JP)
(72) Inventor: HAMAGUCHI, Michinari, Nagoya-shi, Aichi 4580-846 (JP); KOKURYO, Toshio, Nagoya-shi, Aichi 4640-015 (JP); NIMURA, Yuji, 103 Itopia Shimizugaoka, Nagoya-shi, Aichi 4670-038 (JP)
(74) Representative: Wilson Gunn
(86) International application number: PCT/JP2005/011227
(87) International publication number: WO 2006/003804

(56) References cited:
- WO-A-03/062197
- WO-A-2004/033666
- US-A1- 2004 019 003
- FRY ANDREW M: "The Nek2 protein kinase: A novel regulator of centrosome structure" ONCOGENE, BASINGSTOKE, HANTS, GB, vol. 21, no. 40 Review Issue 4, 9 September 2002 (2002-09-09), pages 6184-6194, XP002459977 ISSN: 0950-9232
- HAYWARD DANIEL G ET AL: "The centrosomal kinase Nek2 displays elevated levels of protein expression in human breast cancer" CANCER RESEARCH, vol. 64, no. 20, 15 October 2004 (2004-10-15), pages 7370-7376, XP002463437 ISSN: 0008-5472
- FRY A.M. ET AL: 'A centrosomal function for the human Nek2 protein kinase, a member of the NIMA family of cell cycle regulators.' EMBO.J. vol. 17, no. 2, 1998, pages 470 - 81, XP002991357

### SEQUENCE LISTING

<110> Japan Science and Technology Agency
<120> OLIGORIBONUCLEOTIDE INHIBITING GROWTH OF TUMOR CELLS AND METHOD THEREFOR
<130> FS05-435PCT
<160> 8
<170> PatentIn version 3.1
<210> 1
   <211> 2119
   <212> DNA
   <213> Homo sapiens
<400> 1
   ggcacgagta ggggtggcgg gtcagtgctg ctcgggggct tctccatcca ggtccctgga   60
   gttcctggtc cctggagctc cgcacttggc gcgcaacctg cgtgaggcag cgcgactctg  120
   gcgactggcc ggccatgcct tcccgggctg aggactatga agtgttgtac accattggca  180
   caggctccta cggccgctgc cagaagatcc ggaggaagag tgatggcaag atattagttt  240
   ggaaagaact tgactatggc tccatgacag aagctgagaa acagatgctt gtttctgaag  300
   tgaatttgct tcgtgaactg aaacatccaa acatcgttcg ttactatgat cggattattg  360
   accggaccaa tacaacactg tacattgtaa tggaatattg tgaaggaggg gatctggcta  420
   gtgtaattac aaagggaacc aaggaaaggc aatacttaga tgaagagttt gttcttcgag  480
   tgatgactca gttgactctg gccctgaagg aatgccacag acgaagtgat ggtggtcata 540
   ccgtattgca tcgggatctt aaaccagcca atgttttcct ggatggcaag caaaacgtca 600
   agcttggaga ctttgggcta gctagaatat taaaccatga cacgagtttt gcaaaaacat 660
   ttgttggcac accttattac atgtctcctg aacaaatgaa tcgcatgtcc tacaatgaga 720
   aatcagatat ctggtcattg ggctgcttgc tgtatgagtt atgtgcatta atgcctccat 780
   ttacagcttt tagccagaaa gaactcgctg ggaaaatcag agaaggcaaa ttcaggcgaa 840
   ttccataccg ttactctgat gaattgaatg aaattattac gaggatgtta aacttaaagg 900
   attaccatcg accttctgtt gaagaaattc ttgagaaccc tttaatagca gatttggttg 960
   cagacgagca aagaagaaat cttgagagaa gagggcgaca attaggagag ccagaaaaat 1020
   cgcaggattc cagccctgta ttgagtgagc tgaaactgaa ggaaattcag ttacaggagc 1080
   gagagcgagc tctcaaagca agagaagaaa gattggagca gaaagaacag gagctttgtg 1140
   ttcgtgagag actagcagag gacaaactgg ctagagcaga aaatctgttg aagaactaca 1200
   gcttgctaaa ggaacggaag ttcctgtctc tggcaagtaa tccagaactt cttaatcttc 1260
   catcctcagt aattaagaag aaagttcatt tcagtgggga aagtaaagag aacatcatga 1320
   ggagtgagaa ttctgagagt cagctcacat ctaagtccaa gtgcaaggac ctgaagaaaa 1380
   ggcttcacgc tgcccagctg cgggctcaag ccctgtcaga tattgagaaa aattaccaac 1440
   tgaaaagcag acagatcctg ggcatgcgct agccaggtag agagacacag agctgtgtac 1500
   aggatgtaat attaccaacc tttaaagact gatattcaaa tgctgtagtg ttgaatactt 1560
   ggccccatga gccatgcctt tctgtatagt acacatgata tttcggaatt ggttttactg 1620
   ttcttcagca actattgtac aaaatgttca catttaattt ttctttcttc ttttaagaac 1680
   atattataaa aagaatactt tcttggttgg gcttttaatc ctgtgtgtga ttactagtag 1740
   gaacatgaga tgtgacattc taaatcttgg gagaaaaaat aatattagga aaaaaatatt 1800
   tatgcaggaa gagtagcact cactgaatag ttttaaatga ctgagtggta tgcttacaat 1860
   tgtcatgtct agatttaaat tttaagtctg agattttaaa tgtttttgag cttagaaaac 1920
   ccagttagat gcaatttggt cattaatacc atgacatctt gcttataaat attccattgc 1980
   tctgtagttc aaatctgtta gctttgtgaa aattcatcac tgtgatgttt gtattctttt 2040
   tttttttctg tttaacagaa tatgagctgt ctgtcattta cctacttctt tcccactaaa 2100
   taaaagaatt cttcagtta 2119
<210> 2
   <211> 20
   <212> DNA
   <213> Artificial sequence
<220>
   <223> primer
<400> 2
   aaggaatgcc acagacgaag 20
<210> 3
   <211> 20
   <212> DNA
   <213> Artificial sequence
<220>
   <223> primer
<400> 3
   tgcgattcat ttgttcagga 20
<210> 4
   <211> 20
   <212> DNA
   <213> Artificial sequence
<220>
   <223> primer
<400> 4
   ctctgtgttg gcgtacaggt 20
<210> 5
   <211> 20
   <212> DNA
   <213> Artificial sequence
<220>
   <223> primer
<400> 5 tcatcaccat tggcaatgag 20
<210> 6
   <211> 21
   <212> DNA
   <213> Homo sapiens
<400> 6 21
   aagagggcga caattaggag a 21
<210> 7
   <211> 21
   <212> DNA
   <213> Homo sapiens
<400> 7 21
   aaggaatgcc acagacgaag t 21
<210> 8
   <211> 21
   <212> DNA
   <213> Homo sapiens
<400> 8 21
   aaggagggga tctggctagt g 21

## Description

### Field of the Invention

This invention relates to a method for inhibiting the growth of tumor cells by repressing the expression of NEK2 gene and more particularly to a method for repressing the expression of NEK2 gene by RNA interference method by the use of a specific oligoribonucleotide and inhibiting the growth of tumor cells.

### Prior Art

Signal transduction has been studied as a fundamental mechanism in maintaining cellular function, and abnormality in the pathway of signal transduction has been implicated in various diseases such as cancer. A targeted molecular therapy, whose targeted factor is related to signal transduction, has been paid much attention.

Nek2 is a protein with molecular weight of 51,763Da composed of 445 amino acids, whose coding gene is located on 1q32.2-q41, and has serine threonine kinase or leucine zipper motif and PPI binding site at the C-terminal. Nek2 was identified in mammals by homology search of NIMA gene (Never In Mitosis A), which is identified in Aspergillus and is related to M phase progression.

Nek2 kinase has been studied mainly in relation to cell cycle and chromosome segregation (Reference 3). It is known that Nek2 protein is localized in a centrosome and is highly expressed during late G₂ phase. Therefore, Nek2 might be related to the control of G₂/M phase. Nek2 is functional in division of centrosome in G₂ phase by phosphorylating c-Napl, a physiological substrate, during cell cycle (Reference 4). G₂/M phase during cell cycle is a period of chromosome segregation and cell division as well as division of centrosome. Abnormal Nek2 kinase functional during G₂/M phase could induce aneuploidy by uneven chromosome segregation and is referred to a causal factor of tumorgenesis.

It has been tried to control cell cycle using the NIMA kinase, particularly to enhance or to inhibit physiological function during G₂/M phase (Reference 1). Furthermore, double-stranded DNA corresponding to the nucleotide sequence of NEK2 kinase gene (accession number NM_002497) is known to be used as RNAi, which inhibits Nek2 kinase and prevents growth of tumor cells (Reference 2).

Reference 1: Japanese Patent Application Public Disclosure No. 2003-144168
Reference 2: US2004/0019003 A1
Reference 3: Cell Growth Differ. 5, 625-635, 1992
Reference 4: EMBO J. 17, 470-481, 1998

### Problems to be solved by the Invention

The present inventors found that NEK2 kinase (accession number NM_002497) is expressed specifically in tumor cells such as bile duct carcinoma cells and that repression of the expression of NEK2 kinase by the use of RNA interference method led to inhibition of the growth of the tumor cells.

### Means to solve the Problems

The present inventors found effective RNA sequence in inhibiting the growth of tumor cells such as bile duct carcinoma cells using RNA interference method and accomplished the present invention.

More specifically, the present invention is an oligoribonucleotide (1), an oligoribonucleotide (2) that is complementary to the oligoribonucleotide (1) or a double-stranded RNA comprising the oligoribonucleotides (1) and (2), wherein the oligoribonucleotide (1) corresponds to less than 30 consecutive bases of nucleotide sequence of SEQ ID NO: 1 (NEK2 gene), which includes at least 19 consecutive nucleotides of bases 507-527, 1918-1938, 799-819, 989-1009, 1213-1233 or 1836-1856 of SEQ ID NO: 1.

Furthermore, the present invention is a cytostatic agent containing, as an effective component, the oligoribonucleotide (1), the oligoribonucleotide (2) that is complementary to the oligoribonucleotide (1) or the double-stranded RNA comprising the oligoribonucleotides (1) and (2).

Moreover, the present invention is useful in a method for inhibiting growth of tumor cells. The present invention further comprises the oligoribonucleotide (1), the oligoribonucleotide (2) that is complementary to the oligoribonucleotide (1) or the double-stranded RNA comprising the oligoribonucleotides (1) and (2) for use in inhibiting growth of tumor cells.

Still furthermore, the present invention is a kit for growth inhibition of tumor cells, containing the oligoribonucleotide (1), the oligoribonucleotide (2) that is complementary to the oligoribonucleotide (1) or the double-stranded RNA comprising the oligoribonucleotides (1) and (2).

### Brief Description of the Drawings

Figure 1 shows the microarray examined the gene expression in three kinds of cholangiocellular carcinoma cells (HuCCT1, TFK1, HuH28).
Figure 2 is the Western blotting showing NEK2 expression in five kinds of cholangiocellular carcinoma cells (HuCCT1, TFK1, HuH28, CCKS1, 293).
Figure 3 is the Western blotting showing the repressed expression of NEK2 by each double-stranded RNA in cholangiocellular carcinoma cells (HuCCT1).
Figure 4 shows growth inhibition of cholangiocellular carcinoma cells (HuCCT1) infected with each double-stranded RNA. The ordinate shows absorbance.
Figure 5 are photographs showing the effect of siRNA27 on BalB/c mouse injected with cholangiocellular carcinoma cells (HuCCT1).
Figure 6 shows the effect of siRNA27 on tumor volume of BalB/c mouse injected with cholangiocellular carcinoma cells (HuCCT1).
Figure 7 shows the survival benefit of mouse with bile duct carcinoma administered siRNA27.

### Detailed Description of the Invention

Originally, RNA interference was reported as a method for repressing a specific gene expression by the use of long double-stranded RNA in nematodes. In mammals, long double-stranded RNA induced immunological response and did not induce gene repression. However, it has been demonstrated that small double-stranded RNA (siRNA) induced gene repression in mammals. At present, the size of siRNA mainly used is 19-23 base double-stranded RNA. While more than 30 bases induce immunological response, less than 16 base double-stranded RNA results in increased production of complementary sequence, loses gene specificity for repression, and has a possibility to repress other genes than the target gene.

In the present invention, siRNA with the following features is effective:
1. The initial two bases are AA,
2. The percent composition of G plus C in the sequence is 42 to 58%.

The siRNA of NEK2 gene (SEQ ID NO: 1) satisfying the above two features comprises siRNA having complementary sequence of consecutive 2 bases and having more than 4 base single-stranded RNA at the 3' end in the secondary structure, as shown below. The number at the head of the sequence shows the base number in SEQ ID NO: 1 (NEK2 gene).
507-527 AAGGAATGCCACAGACGAAGT (No of free bases at the 3' end is 11)
1918-1938 AACCCAGTTAGATGCAATTTG (No of free bases at the 3' end is 7)
799-819 AAGAACTCGCTGGGAAAATCA (No of free bases at the 3' end is 6)
989-1009 AAGAGGGCGACAATTAGGAGA (No of free bases at the 3' end is 6)
1213-1233 AACGGAAGTTCCTGTCTCTGG (No of free bases at the 3' end is 6)
1836-1856 AATGACTGAGTGGTATGCTTA (No of free bases at the 3' end is 5)

Since reassociation of the complementary sequences in a molecule may leave free bases at the 3' end, it has been interpreted that the more the number of free bases is, the more effective the activity of repression on NEK2 kinase is by RNA interference. The siRNA of the present invention is an oligoribonucleotide (1), an oligoribonucleotide (2) that is complementary to the oligoribonucleotide (1) or a double-stranded RNA comprising the oligoribonucleotides (1) and (2), wherein the oligoribonucleotide (1) corresponds to less than 30, preferably less than 27, and more preferably less than 23 consecutive bases of nucleotide sequence of SEQ ID NO: 1, which includes at least 19 consecutive nucleotides of bases 507-527, 1918-1938, 799-819, 989-1009, 1213-1233 or 1836-1856 of SEQ ID NO: 1.

Furthermore, it is reported in the Reference 2 that double-stranded RNA corresponding to a part of the sequence of NEK2 kinase (accession number NM_002497) is used as RNAi inhibiting Nek2 kinase and preventing the growth of tumor cells. However, the sequence described in the reference is exhaustive. Although it is described in the example of Reference 2 that the sequence of SEQ ID NO: 3 to SEQ ID NO: 12 of Reference 2, particularly that of SEQ ID NO: 12 is effective, the siRNA of the present invention is selected based on completely different point of view and is different from the sequence of the previous reference. Actually, RNA interference effect of SEQ ID NO: 3 to SEQ ID NO: 12 of Reference 2, in which SEQ ID NO: 12 corresponds to siRNA528 in the Example 1 of the present patent application, is extremely inferior to that of the present invention.

Although RNA fragment used in the present invention could be sense or antisense RNA of the target RNA, these single-stranded RNA might be easily degraded by RNase and be less effective. Therefore, double-stranded RNA composed of the RNA is preferably used. The double-stranded RNA is usually prepared by hybridization of two single-stranded RNA (i.e. sense and antisense RNA) prepared separately.

The oligoribonucleotide "corresponding to" a specific sequence of NEK2 gene means the RNA complementary to a part of mRNA generated after transcription of NEK2 gene, in which the part of mRNA corresponds to the specific sequence of NEK2 gene. Practically, the oligoribonucleotide is obtained by replacing T of the specific DNA sequence of NEK2 gene with U.

The target cells of the present invention are tumor cells of human et al. These tumor cells include biliary tract carcinoma (cholangiocellular carcinoma, gall bladder cancer), breast cancer, pancreatic cancer, esophagus cancer, gastric cancer, colorectal carcinoma, hepatocellular carcinoma, lung cancer, larynx cancer, pharyngeal cancer, thyroid cancer, uterine cancer, ovarian cancer, renal carcinoma, prostate carcinoma, bladder carcinoma, malignant melanoma, brain tumor.

Cytostatic agent of tumor cells may include an oligoribonucleotide, the complementary oligoribonucleotide, or double-stranded RNA composed of these oligoribonucleotides as an effective component, and furthermore may include antisenses, DNA enzymes, peptides, or neutralising antibodies.

Furthermore, the cytostatic agent of tumor cells could be combination with other known cytostatic agents and others. The cytostatic agent of tumor cells of the present invention may be a form of kit containing other agents as described, or may contain such pharmaceutically acceptable medium as sterilized isotonic saline, preservatives, buffer and others.

Moreover, the cytostatic agent of tumor cells of the present invention may provide such kits as injection kit to administer the formulated the cytostatic agent after mixing with diluent and tablet kit to administer individual formulated tablet.

There is no restriction of the method for introducing RNA fragment into cells and the method includes calcium phosphate method, microinjection method, protoplast fusion method, electroporation, and the use of virus vector. Commercially available transfection reagent based on liposome is conveniently used.

The examples of administration of siRNA of the present invention includes the following methods:
(1) As a preoperative administration for biliary tract carcinoma (cholangiocellular carcinoma, gall bladder cancer), (1-1) siRNA dissolved in saline is administrated into hepatic artery under angiography. (1-2) siRNA dissolved in saline or cell matrix^{R} (Nitta Geratin) is administrated directly into bile duct via percutaneous transhepatic biliary drainage (PTCD) tube, or administrated directly into tumor by ultrasound guided endermic paracentesis.
(2) For cancerous peritonitis (peritoneal dissemination) derived from biliary tract carcinoma (cholangiocellular carcinoma, gall bladder cancer), siRNA dissolved in saline or cellmatrix^{R} (Nitta Geratin) is administered directly into peritoneal cavity by abdominal operation or administered endermically into peritoneal cavity under ultrasound guidance.
(3) For inoperable patients with biliary stenosis due to biliary tract carcinoma (cholangiocellular carcinoma, gall bladder cancer), siRNA dissolved in saline or cellmatrix^{R} (Nitta Geratin) is administered directly into bile duct via percutaneous transhepatic biliary drainage (PTCD) tube.

The following Examples illustrate the present invention, but it is not intended to limit the scope of the present invention. In the following Example, five kinds of cholangiocellular carcinoma cell lines (HuCCT1, TFK1, HuH28, CCKS1, 293) were used, and HuCCT1, TFK1 and HuH28 are obtained from Institute of Development, Aging and Cancer, Tohoku University; CCKS1 (cell line established from bile duct carcinoma) is from Pathology Department, Kanazawa University; 293 (cell line established from faetal kidny) is from RIKEN.

### Test Example 1

In this Example, exhaustive gene analysis on three kinds of cholangiocellular carcinoma cell lines (HuCCT1, TFK1, HuH28) and four kinds of clinical samples was performed and the expression of NEK2 was investigated.

Cholangiocellular carcinoma cells are cultured in 10 cm Petri dish up to 80 % confluent. The culture was washed two times with PBS, homogenized with 26G injection needle after addition of 1 ml TRIzol™ (LIFE TECHNOLOGIES) in 10 cm dish and was allowed to rest at room temperature for 30 min. RNA was recovered by extraction with chloroform and by precipitation in isopropyl alcohol according to the manual of TRIzol™.

RNA was added with 1ml of 75% ethanol, centrifuged at 15000rpm for 10 min at 4°C and recovered as a precipitation. RNA was dissolved in 100 µl RNA free water on ice. mRNA was purified by the use of µ MACS mRNA isolation kit™ (Millenyl Biotec). Dissolved RNA was added with 200 µl Lysis/Binding Buffer according to the manual of µ MACS mRNA isolation kit™. It was mixed well, heated at 68°C for 3 min and allowed to rest on ice for 10 min.

Furthermore, 50 µl Oligo (dt) Microbeads was added to the dissolved RNA solution. mRNA was extracted with MACS column Type µ. The yield of RNA was determined by the measurement of absorption at 260 nm and 280 nm. mRNA was precipitated by ethatinmate and stored at -80°C.

In contrast, 1 g excised tissue (normal liver or bile duct carcinoma) was frozen in liquid nitrogen, crushed, added with 1 ml TRIzol™ homogenized with 26G injection needle and was allowed to rest at room temperature for 30 min. RNA extraction and mRNA purification was performed as above described.

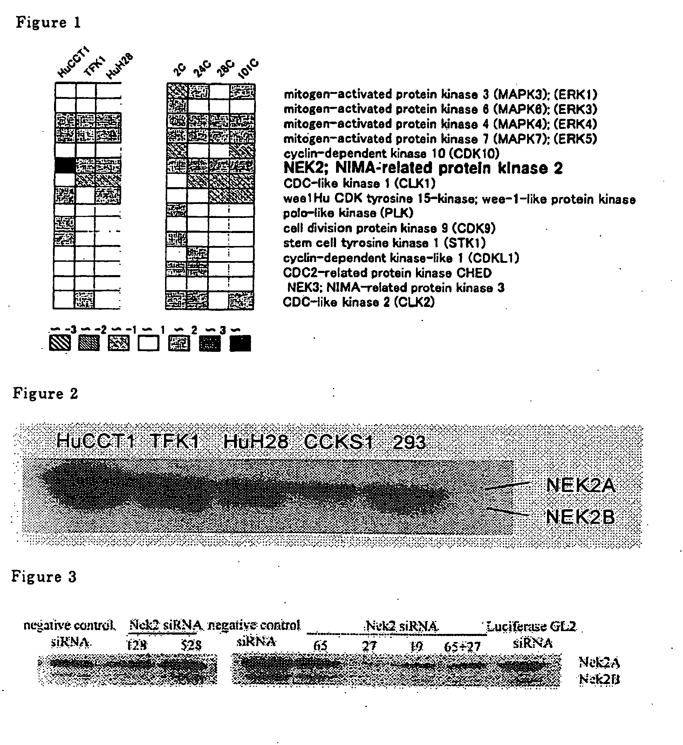

³²P labeled cDNA was prepared from 5 µg mRNA, DNA primer and α³²PdATP by the use of reverse transcriptase according to the manual of. Atlas Human Cancer 1.2 Array™ (CLONTECH). Total five µg mRNA composed of each 1 µg mRNA extracted from normal liver tissue of 5 cases was used as a control. Hybridization was performed for 9 hr at 68°C, after addition of ³²P labeled cDNA onto array membrane after prehybridization. The array membrane was washed with solution 1 for 30 min for three times at 68°C and with solution 2 for 30 min for two times at 68°C. The array membrane was packed in plastic bag and exposed to an image plate at room temperature for 48 hrs. The isotope signal was detected by an image analyzer. For individual gene, total ionizing radiation dose was measured as signal strength (PSL: PSL=αDT, D: radiation dose of RI labeled sample on image plate, T: exposure time, α: constant) and radiation dose per area was calculated based on an image area. The result was shown in Fig. 1.

After the correction of back ground of measured radiation dose per area, correction by β-actin was performed to normalize the expression of house keeping gene among various array membranes. The ratio of signal strength of cholangiocellular carcinoma cells against that of control was expressed by log2. More than 1, which means the ratio of expression is more than two times, is interpreted as enhanced expression and less than -1, which means that the ratio of expression is less than 1/2, is interpreted as repressed expression.

For NEK2, the 6^{th} gene from the above, the expression was more than 1 for all cell lines and clinical samples, i.e. the expression is enhanced. Particularly, for HuCCT1, the remarkably enhanced expression is exhibited.

### Test Example 2

In this Example, the expression of NEK2 was examined for 5 kinds of cholangiocellular carcinoma cells (HuCCT1, TFK1, HuH28, CCKS1, 293) by Western blotting.

First of all, cell lysate of cholangiocellular carcinoma cells was prepared. Each of five kinds of cholangiocellular carcinoma cells was cultured in 10 ml Petri dish up to 80% confluent. The cultures were washed with PBS for two times, added with 300 µl sample buffer, homogenized with 21G injection needle and heated at 96°C for 3 min. Then, a separation gel was prepared. 8.52 ml sterilized distilled water, 11.1 ml of 1 M Tris-HCl (pH 8.8), 300 µl of 10 % SDS, 10 ml bis-acrilamid, 300 µl APS, and 25 µl TEMED were mixed and allowed to rest at room temperature for 30 min.

Later, a stacking gel was prepared. 4.22 ml sterilized distilled water, 0.75 ml of 1 M Tris -HCl (pH 6.8), 60 µl of 10 % SDS, 0.9 ml bis-acrilamid, 60 µl APS, and 10 µl TEMED were mixed, overlaid on a separation gel and allowed to rest at room temperature for 15 min.

After that, 20 µl of each lysate together with makers was electrophoresed for 60 min under CC40A. After the electrophoresis, a transfer membrane was overlaid on the gel, energized at 15 V for 45 min, and transferred the proteins on the gel to the transfer membrane.

In the meantime, 5 % skim milk solution was prepared by adding 50 ml 1 x TBS-T to 2.5 g skim milk. Blocking solution was prepared by putting transfer membrane into 5% skim milk solution and by warming at 37°C for 60 min with constant shaking.

Then, the first antibody solution diluted to 500 fold was prepared by the addition of 500 µl of 5% skim milk to 1 µl first antibody (NEK2: Transduction Laboratories or β-actin: SIGMA) and by mixing. The transfer membrane was added to the first antibody solution and warmed at 37°C for 60 min with constant shaking. The transfer membrane was washed for three times with 1 x TBS-T at room temperature for 10 min.

The second antibody solution diluted to 4000 fold was prepared by the addition of 2 ml of 5% skim milk to 0.5 µl second antibody (Goat F(ab') anti Mouse Ig's HRP conjugated: BIOSOURCE) and by mixing. The transfer membrane was added to the second antibody solution and warmed at 37°C for 60 min with constant shaking. The transfer membrane was washed for three times with 1 x TBS-T at room temperature for 10 min.

ECL™ reaction solution was prepared by the mixture of 500 µl ECLA solution with 500 µl B solution according to a manual of ECL™ solution (PerkinElmer Life Science). Transfer membrane was enclosed in a plastic bag and was added with ECL reaction solution. The transfer membrane was exposed to Roentgen film for two min at room temperature in a dark room. The Roentgen film was developed and NEK2 was detected by chemiluminescence.

The result is shown in Fig.2. NEK2 is composed of NEK2A and NEK2B, wherein the C-terminal of NEKB2 is spliced from NEK2A. The level of expression was compared based on the ratio of NEK2 concentration to the data of β-actin used as a control and HuCCT1 cells were found to express NEK2 the most highly.

In the following Examples, cholangiocellular carcinoma cells (HuCCT1) were used because they expressed the most highly, are transplantable into nude mouse and proliferated the most rapidly.

### <Example 1>

In the Example, repressed expression of NEK2 in cholangiocellular carcinoma cells (HuCCT1) was examined with 5 kinds of double-stranded RNA by the use of Western blotting. The following 5 kinds of siRNA were constructed and prepared.
(1) siRNA 65: Sense strand siRNA (GAGGGCGACAAUUAGGAGATT; SEQ ID NO: 11) and antisense strand siRNA (UCUCCUAAUUGUCGCCCUCtt; SEQ ID NO: 12) corresponding to AAGAGGGCGACAATTAGGAGA (SEQ ID NO: 6) were prepared and used after hybridization.
(2) siRNA 27: Sense strand siRNA (GGAAUGCCACAGACGAAGUtt; SEQ ID NO: 13) and antisense strand siRNA (ACUUCGUCUGUGGCAUUCCtt; SEQ ID NO: 14) corresponding to AAGGAATGCCACAGACGAAGT (SEQ ID NO: 7) were prepared and used after hybridization.
(3) siRNA 19: Sense strand siRNA (GGAGGGGAUCUGGCUAGUGtt; SEQ ID NO: 15) and antisense strand siRNA (CACUAGCCAGAUCCCCUCCtt SEQ ID NO: 16) corresponding to AAGGAGGGGATCTGGCTAGTG (SEQ ID NO: 8) were prepared and used after hybridization.
(4) siRNA65+27: The equal amount of (1) and (2) were mixed and used.
(5) siRNA 128: Sense strand siRNA and antisense strand siRNA corresponding to AGAAAAAATAATATTAGGAAAA (SEQ ID NO: 9) were prepared and used after hybridization.
(6) siRNA 528 : Sense strand siRNA and antisense strand siRNA corresponding to CCTGGATGGCAAGCAAAACGTC (SEQ ID NO: 10) were prepared and used after hybridization.
(7) Luciferase GL2 siRNA: Commercial product (DHARMACON) was used as a control. This is siRNA against Luciferase, which is not present in human body. It affects neither to the expression of NEK2 nor to cell proliferation.

The concentration of each siRNA was prepared at 20 µM and used for the example.

On the other hand, cholangiocellular carcinoma cells (HuCCT1) were cultured in 3.5 cm Petri dish until 60% confluent. The culture was washed two times with the same medium without serum and added with 800 µl of the medium without serum.

4 µl of each 20 µM siRNA and 96 ml medium were mixed. At the same time, 20 µl GenePOTER™ (Gene Therapy System) and 80 µl medium were mixed. 200 µl siRNA GenePOTER™ mixture was prepared by their mixing and allowed to rest for 30 min at room temperature. The above siRNA GenePOTER™ was added to the 3.5 cm culture dish containing cholangiocellular carcinoma cells and medium without serum. It was incubated for 3 hr at 37°C in CO₂ incubator. Then, 1ml of medium containing 20% serum was added to the 3.5 cm dish containing siRNA Gene POTER™ mixture. It was incubated in CO₂ incubator at 37°C for 72 hr. The expression of a protein was examined by the Western blotting described in the Test Example 2. The result is shown in Fig.3. At 72 hr time point, siRNA27 and siRNA19 show the similar repression of NEK2 protein expression. On the other hand, siRNA128 and siRNA528 show similar repression to the control and show no repression effect of NEK2 protein expression.

In addition, siRNA65 show little repression effect at 72 hr time point, however it shows certain repression effect at one week time point (the result is not shown). The action mechanism of siRNA27 may be different from that of siRNA19 and siRNA65.

### <Example 2>

In this Example, proliferation assay was performed for cholangiocellular carcinoma cells (HuCCT1) infected with double-stranded RNA used in Example 1. As a control, the result by the use of the cell lines infected with only GenePORTER™ is shown.

Cholangiocellular carcinoma cells (HuCCT1) were infected with five kinds of double-stranded RNA according to the method shown in Example 1.

The culture was washed with PBS for two times, added with 1ml of 0.1% trypsin/EDTA and incubated in a CO₂ incubator at 37°C for 10 min. The cell number was counted and the cell suspension in the medium was prepared at 1 x 10⁶/ml.

Each group is composed of 10 wells in 96 well plate and each well was seeded with 50 µl cells. The plate was incubated in a CO₂ incubator at 37°C for 24 hr. Each well was added with 50 µl medium and 10 µl TetraColor ONE™ (SEIKAGAKU CORPORATION). It was incubated in a CO₂ incubator at 37°C for 3 hr. The absorbance at 450 nm was measured by a microplate reader.

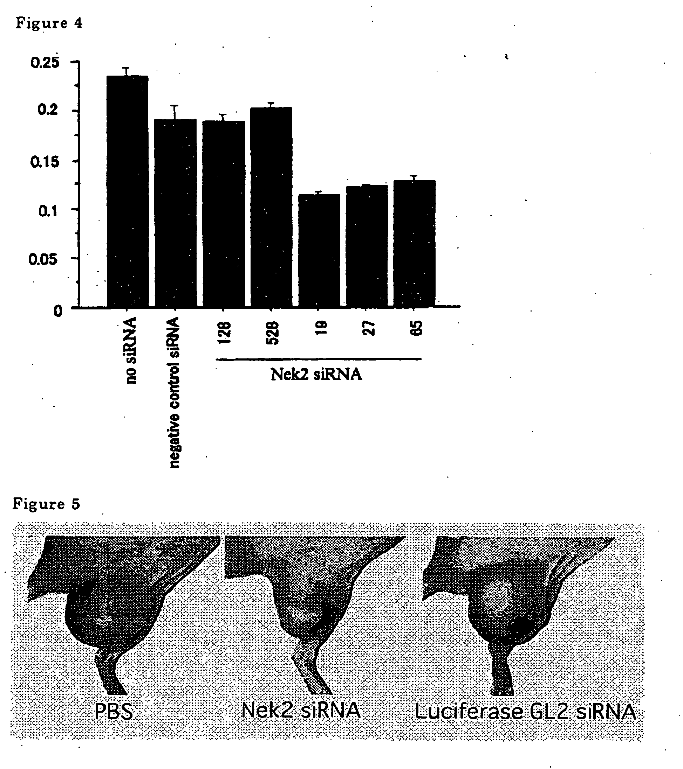

The result is shown in Fig. 4. As in the case of Example 1, siRNA27 and siRNA19 show similar repression effect of expression of NEK2 protein and siRNA 65 shows slight repression effect. In contrast, siRNA 128 and siRNA 528 show similar repression to the control and show no repression effect of NEK2 protein expression.

### <Example 3>

In this Example, effect of siRNA was examined for BalB/c mice inoculated with cholangiocellular carcinoma cells (HuCCT1).

Cholangiocellular carcinoma cells (HuCCT1) were cultured in 15 cm Petri dish until 80% confluent. The culture was washed with PBS for two times, added with 1 ml of 0.1% trypsin/EDTA solution, incubated in a CO₂ incubator at 37°C for 10 min. The culture was washed with PBS for two times. Cell number was counted and the cells were suspended in Hanks™ solution (LIFE TECHNOLOGIES) at 1 x 10⁷/100 µl. Cholangiocellular carcinoma cells (HuCCT1) were injected subcutaneously into a right thigh of an 8 w.o. BalB/c mouse at 1 x 10⁷ cells / 100 µl. The mouse was farmed for a month after the injection.

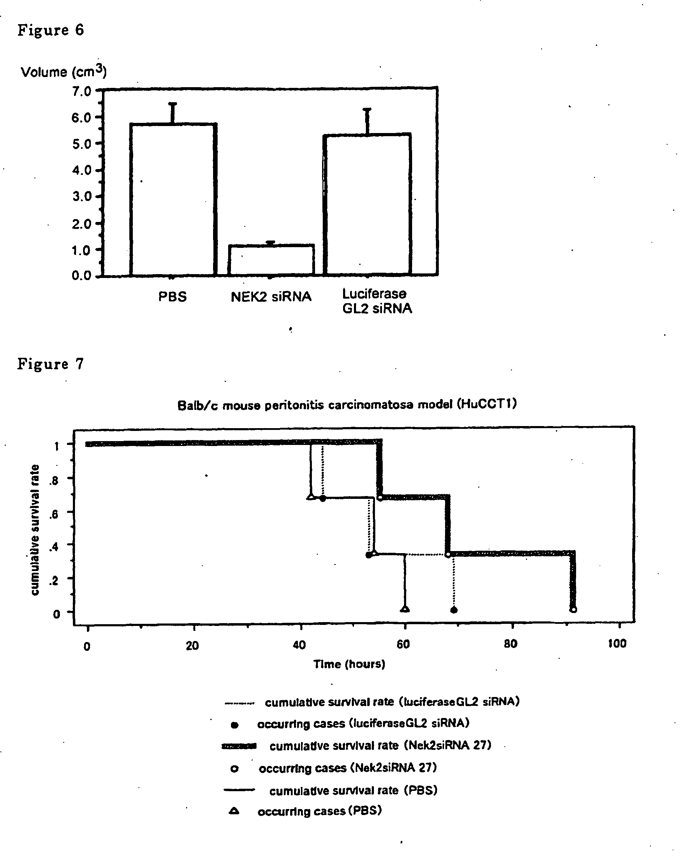

100 µl of 20 µM siRNA 27 was directly injected subcutaneously into tumor in the right thigh. Injection was performed once a week for total three times. After one month of the third siRNA 27 injection, the tumor was exsected and the volume of the tumor was measured and is shown in Fig. 5. Figure 6 shows that size of the tumor decreases according to the administration of siRNA 27.

### <Example 4>

Bile duct carcinoma was injected into mouse peritoneal cavity. After peritoneal dissemination, siRNA 27 was administrated and the survival benefit of the mouse was examined. The result is shown in Fig. 7. Unquestionably, survival benefit was observed.

The oligoribonucleotide of the present invention could be used as a cytostatic agent of tumor cells. Furthermore, the oligoribonucleotide may possibly inhibit growth of inflammatory cells and could be used as preventive medicine for keloidosis, which is induced at the time of operation wound healing.

## Claims

1. An oligoribonucleotide (1), an oligoribonucleotide (2) that is complementary to the oligoribonucleotide (1) or a double-stranded RNA comprising the oligoribonucleotides (1) and (2), wherein the oligoribonucleotide (1) corresponds to less than 30 consecutive bases of nucleotide sequence of SEQ ID NO: 1(NEK2 gene), which includes at least 19 consecutive nucleotides of bases 507-527, 1918-1938, 799-819, 989-1009, 1213-1233 1836-1856 or 403-423 of SEQ ID NO: 1.

2. An oligoribonucleotide (1), an oligoribonucleotide (2) that is complementary to the oligoribonucleotide (1) or a double-stranded RNA comprising the oligoribonucleotides (1) and (2), wherein the oligoribonucleotide (1) corresponds to less than 30 consecutive bases of nucleotide sequence of SEQ ID NO: 1(NEK2 gene), which includes at least 19 consecutive nucleotides of bases 403-423, 507-527 or 989-1009 of SEQ ID NO: 1.

3. A cytostatic agent containing, as an effective component, the oligoribonucleotide (1), the oligoribonucleotide (2) that is complementary to the oligoribonucleotide (1) or the double-stranded RNA comprising the oligoribonucleotides (1) and (2) of either of claims 1 or 2.

4. The oligoribonucleotide (1), the oligoribonucleotide (2) that is complementary to the oligoribonucleotide (1) or the double-stranded RNA comprising the oligoribonucleotides (1) and (2) of either of claims 1 or 2, for use in a method of inhibiting growth of tumor cells.

5. A kit for growth inhibition of tumor cells, containing the oligoribonucleotide (1), the oligoribonucleotide (2) that is complementary to the oligoribonucleotide (1) or the double-stranded RNA comprising the oligoribonucleotides (1) and (2) of either of claims 1 or 2.

## Patentansprüche

1. Oligoribonucleotid (1), Oligoribonucleotid (2), welches zu dem Oligoribonucleotid (1) komplementär ist, oder eine doppelsträngige RNA umfassend die Oligoribonucleotide (1) und (2), wobei das Oligoribonucleotid (1) weniger als 30 aufeinanderfolgenden Basen der Nucleotidsequenz von SEQ ID NO: 1 (NEK2-Gen) entspricht, welches wenigstens 19 aufeinanderfolgende Nucleotide der Basen 507-527, 1918-1938, 799-819, 989-1009, 1213-1233, 1836-1856 oder 403-423 von SEQ ID NO: 1 einschließt.

2. Oligoribonucleotid (1), Oligoribonucleotid (2), welches zu dem Oligoribonucleotid (1) komplementär ist, oder eine doppelsträngige RNA umfassend die Oligoribonucleotide (1) und (2), wobei das Oligoribonucleotid (1) weniger als 30 aufeinanderfolgenden Basen der Nucleotidsequenz von SEQ ID NO: 1 (NEK2-Gen) entspricht, welches wenigstens 19 aufeinanderfolgende Nucleotide der Basen 403-423, 507-527 oder 989-1009 von SEQ ID NO: 1 einschließt.

3. Zytostatikum enthaltend, als wirksame Komponente, das Oligoribonucleotid (1), das Oligoribonucleotid (2), welches zu dem Oligoribonucleotid (1) komplementär ist, oder die doppelsträngige RNA umfassend die Oligoribonucleotide (1) und (2) nach den Ansprüchen 1 oder 2.

4. Oligoribonucleotid (1), Oligoribonucleotid (2), welches zu dem Oligoribonucleotid (1) komplementär ist, oder die doppelsträngige RNA umfassend die Oligoribonucleotide (1) und (2) nach den Ansprüchen 1 oder 2, zur Verwendung in einem Verfahren zum Hemmen des Wachstums von Tumorzellen.

5. Kit zur Hemmung des Wachstums von Tumorzellen, enthaltend das Oligoribonucleotid (1), das Oligoribonucleotid (2), welches zu dem Oligoribonucleotid (1) komplementär ist, oder die doppelsträngige RNA umfassend die Oligoribonucleotide (1) und (2) nach den Ansprüchen 1 oder 2.

## Revendications

1. Oligoribonucléotide (1), oligoribonucléotide (2) qui est complémentaire de l'oligoribonucléotide (1) ou ARN double brin comprenant les oligoribonucléotides (1) et (2), dans lequel l'oligoribonucléotide (1) correspond à moins de 30 bases consécutives d'une séquence nucléotidique de SEQ ID N° 1 (gène NEK2), qui inclut au moins 19 nucléotides consécutifs des bases 507-527, 1918-1938, 799-819, 989-1009, 1213-1233, 1836-1856 ou 403-423 de SEQ ID N° 1.

2. Oligoribonucléotide (1), oligoribonucléotide (2) qui est complémentaire de l'oligoribonucléotide (1) ou ARN double brin comprenant les oligoribonucléotides (1) et (2), l'oligoribonucléotide (1) correspondant à moins de 30 bases consécutives d'une séquence nucléotidique de SEQ ID N° 1 (gène NEK2), qui inclut au moins 19 nucléotides consécutifs des bases 403-423, 507-527 ou 989-1009 de SEQ ID N° 1.

3. Agent cytostatique contenant, en tant que composant efficace, l'oligoribonucléotide (1), l'oligoribonucléotide (2) qui est complémentaire de l'oligoribonucléotide (1) ou ARN double brin comprenant les oligoribonucléotides (1) et (2) selon l'une des revendications 1 ou 2.

4. Oligoribonucléotide (1), oligoribonucléotide (2) qui est complémentaire de l'oligoribonucléotide (1) ou ARN double brin comprenant les oligoribonucléotides (1) et (2) selon l'une des revendications 1 ou 2, pour utilisation dans un procédé d'inhibition de la croissance de cellules tumorales.

5. Ensemble pour l'inhibition de la croissance de cellules tumorales, contenant l'oligoribonucléotide (1), l'oligoribonucléotide (2) qui est complémentaire de l'oligoribonucléotide (1) ou l'ARN double brin comprenant les oligoribonucléotides (1) et (2) selon l'une des revendications 1 ou 2.
